# EUROPEAN PATENT SPECIFICATION

(11) **EP 1 839 584 B1**
(45) Date of publication and mention of the grant of the patent: **07.10.2009**
(21) Application number: 07251364.1
(22) Date of filing: 29.03.2007
(51) Int. Cl.: A61B 17/04, A61F 2/08

(54) **Deployment stabilization unit for a bone anchoring device**
Stabilisierungseinheit für die Anbringung einer Knochenverankerungsvorrichtung
Unité de stabilisation pour le déploiement d'un dispositif d'ancrage d'os

(30) Priority: 30.03.2006 US 393504
(43) Date of publication of application: 03.10.2007
(73) Proprietor: ETHICON, INC., Somerville, New Jersey 08876 (US)
(72) Inventor: Li, Zhigang, Hillsborough, NJ 08844 (US); Cooper, Kevin, Flemington, NJ 08822 (US); Shissias, Raymond, Iselin, NJ 08830 (US); Pellegrino, Richard, Upton, MA 01568 (US); Lasota, Douglas R., Saugus, MA 01906 (US); Fanger, Jonathan, Fall River, MA 02720 (US)
(74) Representative: Fisher, Adrian John

(56) References cited:
- EP-A- 1 199 035
- EP-A- 1 348 380
- WO-A-98/18409
- WO-A-98/38938
- US-B1- 6 562 044

## Description

### FIELD OF THE INVENTION

The present invention relates to a deployment stabilization device unit for bone anchoring devices. More specifically, the invention relates to a deployment stabilization device unit for an anchor for securing a cable within a hole or opening in a bone, the cable being attached to the anchor.

### BACKGROUND OF THE INVENTION

A wide variety of techniques are available to surgeons for securing sutures or cables within a hole or opening in a bone. Conventionally, such an opening may be a bone tunnel with open ends or a bore hole with one open end and an opposed closed end. Screws, rivets, vertebral straps, suture anchors, and other types of interference fitting anchors are commonly used. In all cases a solid and secure attachment between the suture, or cable, and the anchoring member for anchor devices is key.

The prior art is replete with implantable bone fasteners that include screw members having a threaded body. Typically, the cable, or suture, may be directly attached to the screw member in a variety of conventional manners including threading the suture through an internal channel in the body of the screw or threading the suture through an attached eyelet. One type of known bone fastener includes an expandable member having an axial channel and an elongated insertion element insertable therein. When the insertion element is driven into the axial channel in the expandable member, an interference or interlocking fit secures the insertion element to the expander, thereby securing the suture within the bone bore hole. Load forces exerted on the suture act directly on the insertion element so that the security of the suture within the bone bore hole depends on the security of the engagement between the insertion element and the expandable member.

In one known device, an expandable sheath in which the cable load (bearing force) acts directly on an expandable sheath that is expanded by an expander member for an interference fit within a bore hole in a bone. In such a device the cable load (bearing force) acts directly on the expandable sheath so that the fastening strength of the anchor is independent of the axial security of engagement between the expander member and the expandable sheath. Such a bone anchoring device avoids the failure mode of expander separation from the expandable sheath under suture loading.

In general, methods to attach or secure cables to anchoring members are insert molding, or passing the cables through eyelets or small holes in the anchoring members. The disadvantage of the former methods is low pullout strength of the cable from the anchoring member because insert-molding processes cannot form a secure attachment between the cable and anchoring device. In the latter method, the hole or eyelet is related to the removal of material from the anchoring member that may result in mechanical strength lost, or it may be difficult or not possible to place a hole or eyelet at the anchoring member due to a low profile configuration or limited space. Thus, there is a need for a device where the cable can be securely attached without compromising the structural integrity of the anchoring member.

Anchoring devices of the above nature are known from WO 98/18409, US 6562044, EP 1348380 and WO 98/38938. The latter document forms the basis for the preamble of claim 1 appended hereto.

### SUMMARY OF THE INVENTION

The problems and disadvantages of the prior art devices described above are overcome by the present invention through the provision of a novel deployment stabilization unit for a bone anchoring device, as defined in claim 1. Preferably the bone anchoring device has an anchor member, a cable, and a cover. The anchor member is provided with a series of engagement ribs to form an interference fit with the surface of a bone hole and pins for attaching the cable. The cable is attached to the anchor member by penetrating the cable using the pins and securing it in place by welding. A cover may be mounted to the anchoring member, and optionally the cover and pins are welded together.

These and other aspects and advantages of the present invention will become more apparent by the following description and accompanying drawings.

### BRIEF DESCRIPTION OF THE DRAWINGS

FIG. 1A is an exploded perspective view of a bone anchoring device;
FIG. 1B is a perspective view of the bone anchoring device of FIG. 1A, wherein the device is partially assembled;
FIG. 1C is a perspective view of the bone anchoring device of FIG. 1A, wherein the device is fully assembled;
FIG. 2A is a perspective view of an alternate anchoring member;
FIG. 2B is a perspective view of a partially assembled bone anchoring device using the anchoring member of FIG. 2B;
FIG. 2C is a perspective view of the fully assembled bone anchoring device of FIGS. 2A and 2B having an expander in position for expansion;
FIG. 3A is a cross-sectional view of the anchoring device of FIG. 2C prior to deployment in a bone bore hole opening;
FIG. 3B is a cross-sectional view of the device of FIG. 2C, the device being shown in a deployed state within the bone bore hole;
FIG. 4 is a perspective view of a deployment stabilizer unit according to the present invention;
FIG. 5A is a perspective view of a bone-anchoring device assembled with the deployment stabilizer unit of FIG. 4 with an expander in position; and
FIG. 5B is a perspective view of a bone anchoring device fully assembled with the deployment stabilizer unit of FIG. 4 with an expander in position.

### DETAILED DESCRIPTION OF THE INVENTION

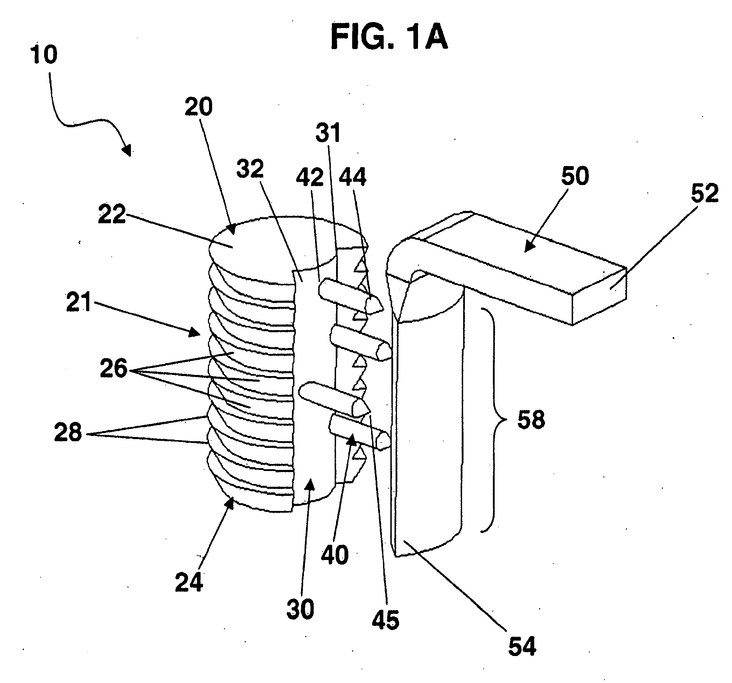

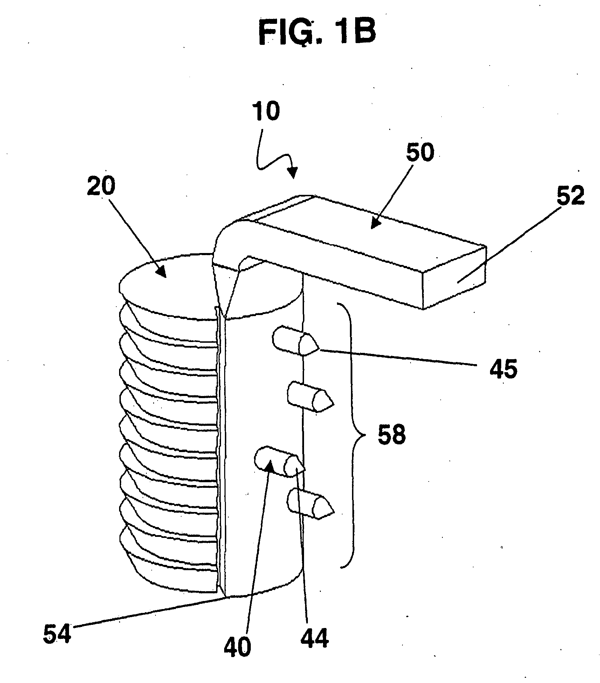

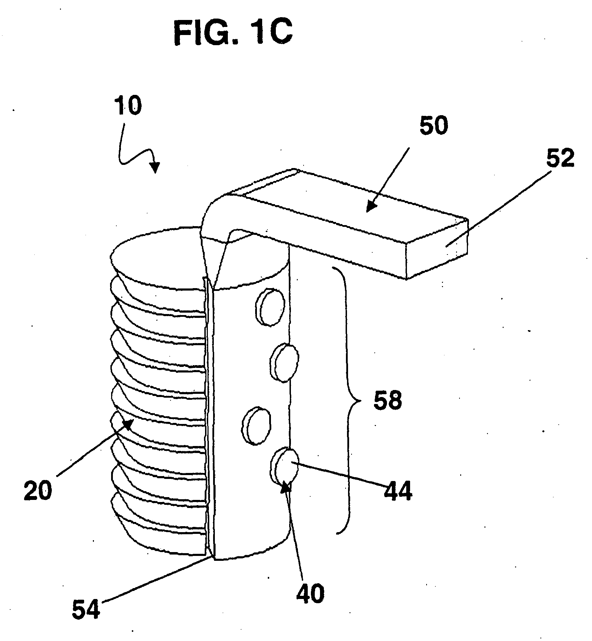

Referring to FIGS. 1A, 1B and 1C there is shown a bone anchoring device 10 for use in surgical procedures in the securing of a cable member **50** to a bone of a patient. The term cable member as used herein refers to a long, generally flat structure such as a solid or perforated polymer ribbon, or a textile structure such as a braided or woven ribbon.

The bone anchoring device **10** includes an anchor member **20** and a cable member **50.** Anchor member **20** is seen to have proximal end **22,** distal end **24,** engagement ribs **26** extending outward from anchor member surface **21** and axial cutout **30** extending into surface **21.** Engagement ribs **26** further include bone engagement edges **28.** The axial cutout **30** further includes bottom **31** adjacent to cutout surface **32** and at least one pin **40** that extends radially outward from the cutout surface **32.** Preferably, and as shown in FIG. 1, a plurality of pins **40** extend radially outward from the cutout surface **32.**

Though pins **40** preferably extend perpendicularly from cutout surface **32,** one skilled in the art, will appreciate that pins **40** may extend at any angle from cutout surface **32.** Though pins **40** are shown in FIG. 1A as randomly located on cutout surface **32,** pins **40** may be aligned on cutout surface **32** in a particular pattern or configuration.

Each pin **40** is seen to have a proximal end **42** and a distal end **44.** The distal ends are seen to have optional pointed piercing tips **45,** but the tips may have any configuration including blunt, rounded, squared-off, and the like. Cable **50** is seen to have proximal end **52** and distal end **54.** In the embodiment shown in FIG. 1 cable **50** is tapered, going from thinner at the distal end **54** to thicker at proximal end **52.** As shown in FIGS. 1B and 1C, cable member **50** is secured to anchor **20** by penetrating cable **50** with pins **40,** i.e., moving the pins **40** through cable **50,** such that a section **58** of cable **50** is substantially contained within cutout **30** and substantially in contact with surface **32** as shown in FIG. 1B. Prior to penetration, cable **50** is oriented with anchor **20** such that distal end **54** of cable **50** aligns with distal end **24** of anchor **20,** and such that proximal end **52** of cable **50** extends beyond proximal end **22** of anchor **20.** Cable **50** and anchor **20** are secured in a variety of conventional manners, preferably by welding pins **40** to cable **50,** resulting in the deformation of the distal ends **44** of pins **40** as shown in FIG. 1C. The bone anchoring devices **10** of the present invention eliminates or greatly minimizes any incidence of anchor failure due to low pullout strength of the cable from the anchor. Additionally, in another embodiment of the current invention (not shown), the anchor **20** and cable **50** are further secured with a cover mounted flush over cable **50.** This offers the further advantage that cable **50** is protected from damage by bone during the insertion process by the cover.

Cable member **50** may be tipped as described below to reinforce the section of cable member **50** attached to anchor member **20** of bone anchoring device **10.** The purpose of tipping cable member **50** is to encapsulate the end of the cable with a material, such as a polymer, for a predetermined length, typically as long as the section to be attached to the anchor member **20.** During the tipping operation, which can be done by several methods including compression and injection molding, holes for pins **40** on anchor member **20** are incorporated. This is done by having posts in the tipping mold to create the holes in cable member **50.** During the tipping process these holes are permanently formed in cable member **50.** The tipping process may strengthen the cable member **50** to help eliminate "cheese wire" damage to cable member **50** under axial loads.

The bone anchoring devices **10** may be used to secure suture or cable within a bone bore hole for a variety of uses in surgical procedures. Typically, the surgeon will form a pilot hole for device **10** using a conventional surgical drill. The diameter of the pilot hole is preferably slightly smaller than the diameter of anchoring device **10.** Device **10** would then be deployed in the pilot hole through the application of force resulting in the device being maintained in the pilot hole via an interference fit.

The anchoring devices of the present invention have a variety of uses in surgical procedures. These uses include reattachment of ligaments or tendons to bone. Optionally, cable member **50** of bone anchoring device **10** could be connected to a second bone anchor (not shown), which would be secured within a second bore hole in bone. This arrangement could be used, for example, to hold a bone block between adjacent vertebrae in spinal fusion procedures.

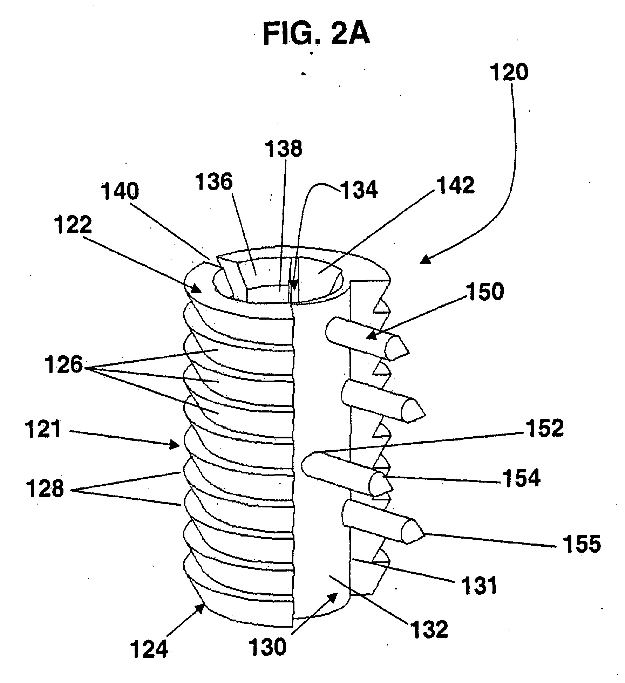

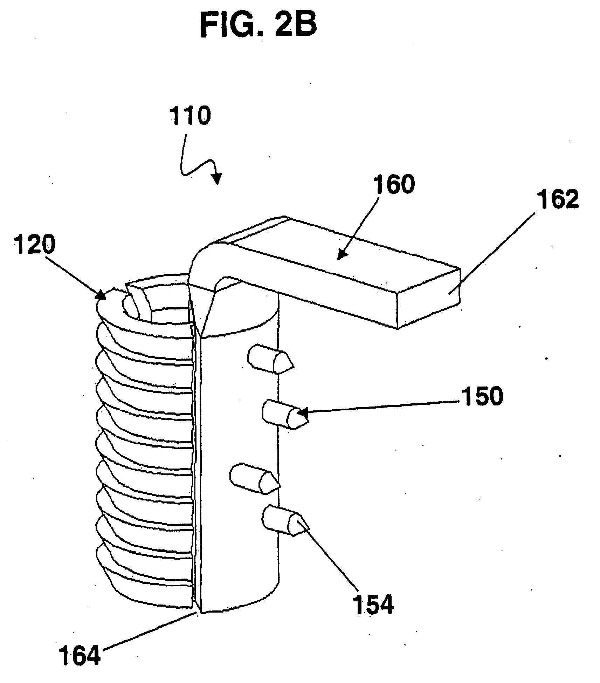

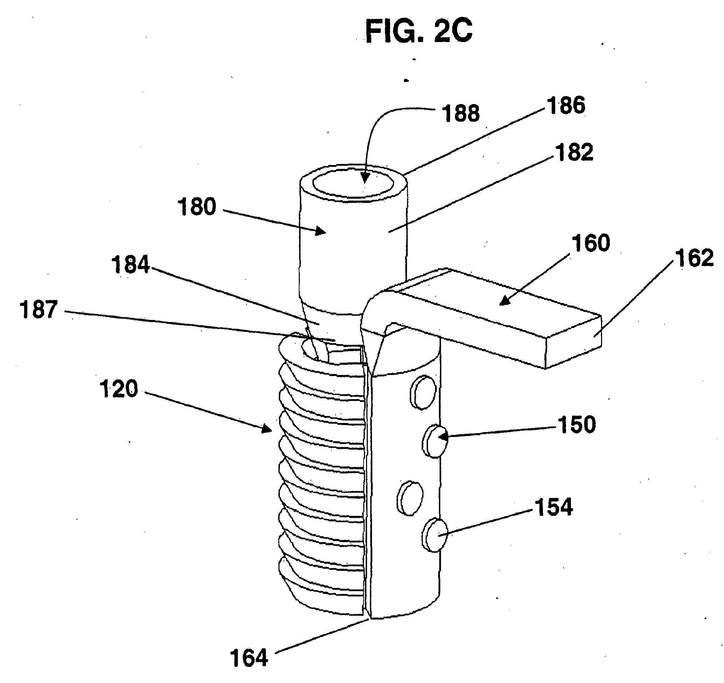

Referring to FIGS. 2A, 2B and 2C, an alternative device is shown having an expandable anchor member **120.** Expandable anchor member **120** is seen to have proximal end **122,** distal end **124,** engagement ribs **126** extending out from surface **121,** axial cutout **130,** axial passageway **134,** chamfered or beveled edge **136,** inner wall surface **138** and axial slot **140.** Axial slot **140** has width W. The engagement ribs **126** have engagement edges **128.** The axial cutout **130** extending into surface **121** is seen to have bottom **131** adjacent to surface **132.** A plurality of pins **150** is seen extending radially outward from the cutout surface **132,** preferably perpendicularly from cutout surface **132.** Pins **150** are seen to have proximal ends **152** and distal ends **154.** Each pin **150** is seen to have distal piercing tip **155,** but the tip **150** may have any configuration including blunt, rounded, squared-off, and the like.

Optionally, expandable anchor member **120** contains a wall hinge to aid in the expansion of expandable anchor member **120.** Preferably, the wall hinge is an inner wall hinge **142** (see FIG. 2A).

As shown in FIGS. 2B and 2C, cable member **160** is secured to expandable anchor member **120** by penetrating cable **160** with pins **150** i.e., moving the pins **150** through the cable **160,** such that cable **160** is substantially contained in cutout **130** and substantially in contact with cutout surface **132:** Prior to penetration, cable **160** is oriented with expandable anchor **120** such that distal end **164** of cable **160** aligns with distal end **124** of anchor member **120,** and such that proximal end **162** of cable **160** extends beyond proximal end **122** of anchor member **120.** Cable **160** and expandable anchor member **120** may be secured together in a variety of conventional ways, including welding pins **150** to cable **160,** resulting in the deformation of distal ends **154** of pins **150** as seen in FIG. 2C. This combination of elements results in an assembled bone anchoring device **110** in which anchor failure due to low pullout strength of the cable is greatly minimized or prevented. In an additional device (not shown), expandable anchor member **120** and cable **160** are further secured with a cover mounted flush over cable **160.** This offers the further advantage that cable **160** is protected from damage by bone during the insertion process by the cover.

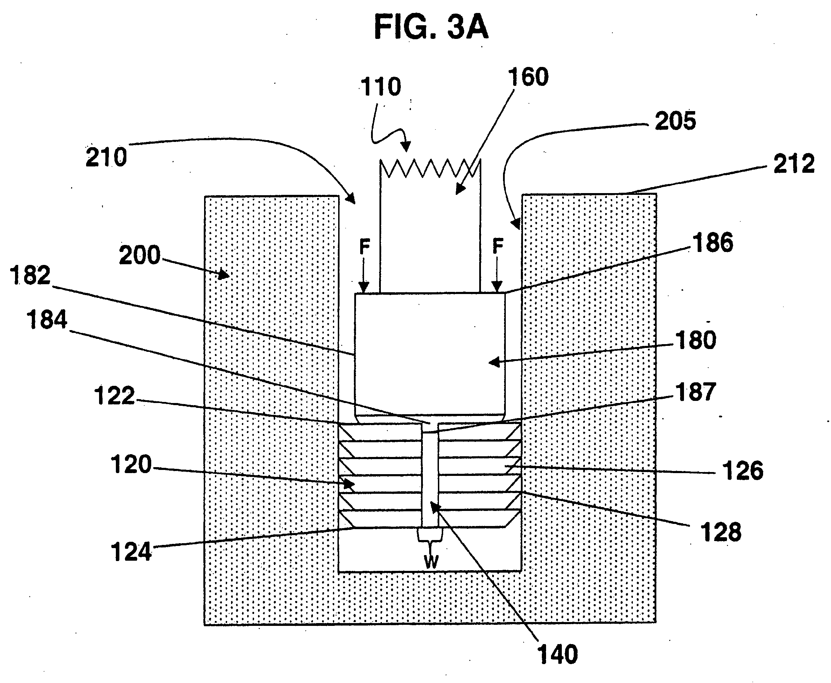

FIG. 2C shows a fully assembled bone-anchoring device **110** with expander member **180** in deployment position. Expander member **180** is generally cylindrically-shaped and includes an outer wall surface **182,** a proximal end **186** and a distal end **187.** The expander member **180** also includes a chamfered or beveled edge **184** located at the distal end **187 of** the expander member **180.** The chamfered edge **184** is used to serve as a lead-in for the initial insertion of the expander member **180** into the expandable anchor **120** as shown in FIG 3A.

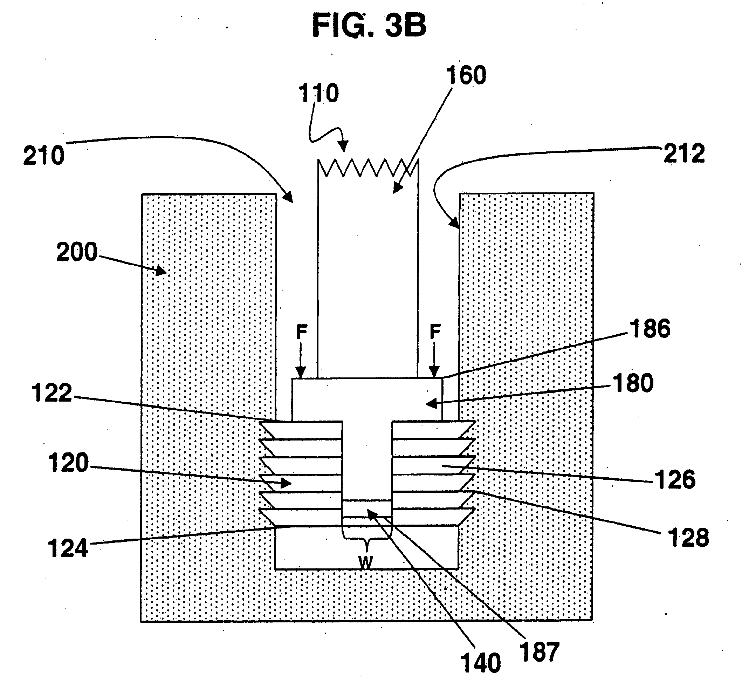

FIGS. 3A and 3B demonstrate the expansion of the bone anchoring device **110** for an interference fit in a bone bore hole. This expansion of the bone anchoring device **110** is achieved by the inward driving of the expander member **180** by a force F into the axial passageway **134** of the expandable anchor member **120.** The interference fit between the outer wall surface **182** of expander member **180** and the inner wall surface **138** of the axial passageway **134** of anchor member **120** forces the anchor **120** to expand radially to conform the inner diameter of passageway **134** to the outer diameter of expander member **180.** The expandable anchor member **120** expands radially due to the further separation of slot **140** with the full insertion of the expander member **180** within the axial passageway **134** of the expandable anchor member **120.** When the bone anchoring device **110** is fully deployed, as shown in FIG. 3B, slot **140** has increased in width W.

FIGS. 3A and 3B show the initial and final configurations, respectively, of the bone anchoring device **110** when deployed in a bone bore hole **210** in order to anchor the cable member **160** to bone **200.**

FIG. 3A shows the initial deployment configuration of the bone anchoring device **110,** which demonstrates the placement of the expander member **180** and the expandable anchor **120** within the bone bore hole **210.** The diameter of the bone bore hole **210** is equal to or only slightly larger than the outer diameter of bone anchoring device **110.** The bone anchoring device **110** is placed within the bone hole **210** such that the proximal surface end **186** of the expander member **180** is flush or below surface **212** of bone **200** (see FIG. 3A). The chamfered edge **184** of the expander member **180** is in contact with the chamfered edge **136** of the anchor **120** in the initial configuration (expander member **180** has not been deployed within anchor **120).** The expander member **180** is then forcibly driven into the axial passageway **134** of the expandable anchor member **120** until full deployment is achieved as shown in FIG. 3B.

With reference to FIG. 3B, the bone anchoring device **110** is shown in its full deployment and final configuration. The expandable anchor **1.20** is shown expanded to a diameter to interfere with or engage the interior bone surface **205** of the bone bore hole **210** allowing the engagement edges **128** of ribs **126** to engage and cut into bone **200.** FIG. 3B also shows the increasing of width W of slot **140** when deployment of the expander member **180** within the expandable anchor **120** is completed. The expansion of slot **140** allows uninhibited circumferential expansion of the expandable anchor **120** for accommodating the circumference of the expander member **180.** The advantage of slot **140** is that it provides for the uniform radial expansion along the entire length of expandable anchor **120** and thus, allows for relatively very large radial and elastic expansion of anchor **120.**

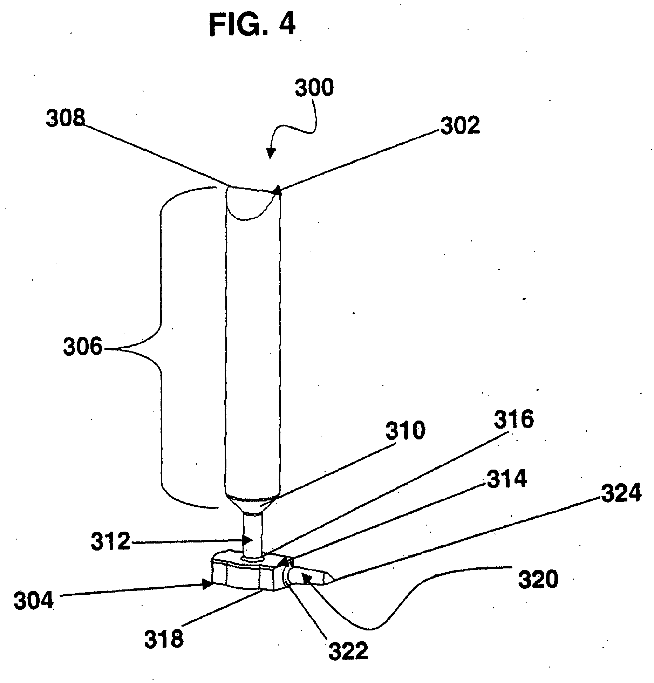

Deployment stabilization unit (DSU) **300** is seen in FIG. 4. DSU **300** contains unit proximal end **302,** unit distal end **304,** post **306,** frangible break-away section **312,** and stabilization seat **314.** Post **306** further contains post proximal end **308** and post distal end **310.** Additionally, stabilization seat **314** contains seat proximal end **316,** seat distal end **318** and seat attachment pin **320** with seat pin proximal end **322** and seat pin distal end **324.**

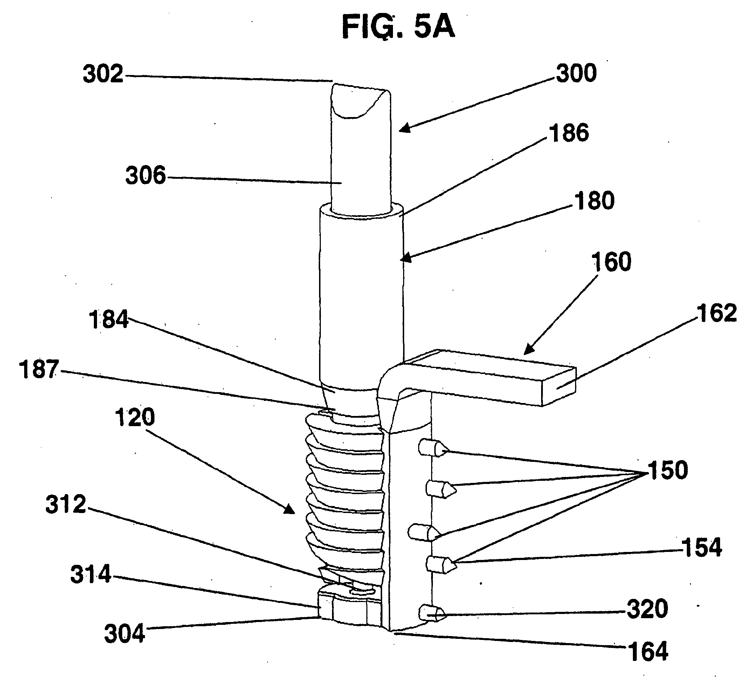

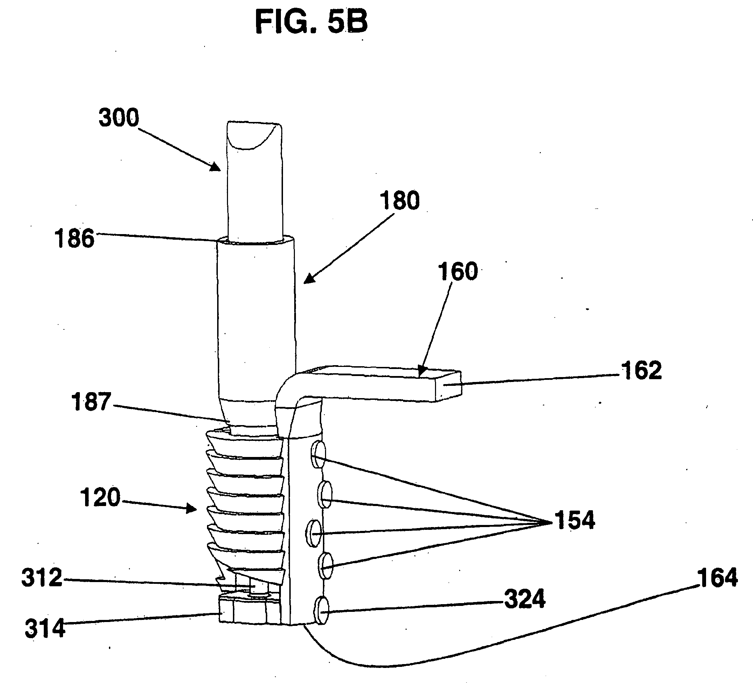

Bone anchoring device **110,** is shown in combination with expander **180** and DSU **300** in FIG. 5A and 5B. DSU **300** is inserted through anchor member **120** by way of axial passageway **134** such that DSU seat proximal end **316** is flush with expandable anchor distal end **124,** and that expandable anchor pins **150** are aligned with seat attachment pin **320.**

As shown in FIGS. 5A and 5B, cable member **160** is secured to expandable anchor member **120** and DSU **300** by penetrating cable **160** with anchor pins **150** and seat pin **320** from pins distal ends **154** and **324** to pins proximal ends **152** and **322,** such that cable **160** is flush with cutout surface **132.** Prior to penetration, cable **160** is oriented such that cable distal end **164** aligns with DSU distal end **304,** and that cable proximal end **162** extends beyond anchor proximal end **122.** Cable **160,** expandable anchor **120** and DSU **300** are secured by the welding of anchor pins **150** and seat attachment pin **320** to cable **160,** resulting in the deformation of pins distal ends **154** and **324** shown in FIG. 5B. This results in bone anchoring device **110** in which anchor failure due to low pullout strength of the cable is substantially eliminated. Additionally, in an alternate embodiment of the current invention (not shown) where anchor **120** and cable **160** are further secured with a cover mounted flush over cable **160** and anchor **120.** This offers the further advantage that cable **160** is protected from damage by bone during the insertion process by the cover.

Use of DSU **300** in combination with bone anchoring device **110** is advantageous because the forces on DSU **300** counter the forces on the expander element (see FIG. 3A and 3B), thus ensuring that bone anchoring device **110** does not migrate in the bone hole **210.** After deployment DSU post **306** of DSU **300** is removed. This is accomplished though the breakage of DSU break-away section **312** either during expander **180** deployment or immediately there after by applying torque to DSU post **306** (not shown).

Uses of bone anchoring device **110** include reattachment of ligaments or tendons to bone. Furthermore, cable member **160** of bone anchoring device **110** could be connected to a second bone anchoring device (not shown), which is secured within a second hole opening in bone. This arrangement could be used, for example, to hold a bone block between adjacent vertebrae in spinal fusion procedures.

Bone anchoring device **110,** expander **180** and deployment stabilization unit (DSU) **300** may be available separately, or can be in combination as part of a bone anchoring kit. The pieces may be fully, or partially, assembled prior to use, and preferably packaged and sterilized prior to being made available.

Suitable materials from which bone anchoring devices **10** and **110** may be formed include biocompatible polymers such as aliphatic polyesters, polyorthoesters, polyanhydrides, polycarbonates, polyurethanes, polyamides and polyalkylene oxides. The present invention also can be formed from absorbable glasses or ceramics comprising calcium phosphates and other biocompatible metal oxides (i.e., CaO), metals, combinations of metals, autograft, allograft, or xenograft bone tissues.

In the preferred embodiment, the bone anchoring device is formed from aliphatic polyester polymers, copolymers or blends thereof. The aliphatic polyesters are typically synthesized in a ring opening polymerization. Suitable monomers include but are not limited to lactic acid, lactide (including L-, D-, meso and D,L mixtures), glycolic acid, glycolide, epsilon-caprolactone, p-dioxanone (1,4-dioxan-2- one), trimethylene carbonate (1,3-dioxan-2-one), delta-valerolactone, beta-butyrolactone, epsilon-decalactone, 2,5-diketomorpholine, pivalolactone, alpha-diethylpropiolactone, ethylene carbonate, ethylene oxalate, 3-methyl-1,4-dioxane-2,5-dione, 3,3-diethyl-1,4-dioxan- 2,5-dione, gamma-butyrolactone, 1,4-dioxepan-2-one, 1,5-dioxepan-2-one, 6, 6-dimethyl-dioxepan-2-one, 6,8-dioxabicycloctane-7-one and combinations thereof. These monomers generally are polymerized in the presence of an organometallic catalyst and an initiator at elevated temperatures. The organometallic catalyst is preferably tin based, e.g., stannous octoate, and is present in the monomer mixture at a molar ratio of monomer to catalyst ranging from about 10,000/1 to about 100,000/1. The initiator is typically an alkanol (including diols and polyols), a glycol, a hydroxyacid, or an amine, and is present in the monomer mixture at a molar ratio of monomer to initiator ranging from about 100/1 to about 5000/1. The polymerization typically is carried out at a temperature range from about 80°C. to about 240°C., preferably from about 100°C. to about 220°C., until the desired molecular weight and viscosity are achieved.

In another embodiment of the present invention, the polymers and blends can be used as a therapeutic agent release matrix. Prior to forming the bone anchoring device, the polymer would be mixed with a therapeutic agent. The variety of different therapeutic agents that can be used in conjunction with the polymers of the present invention is vast. In general, therapeutic agents which may be administered via the pharmaceutical compositions of the invention include, without limitation: anti-infectives such as antibiotics and antiviral agents; chemotherapeutic agents (i.e. anticancer agents); antirejection agents; analgesics and analgesic combinations; anti-inflammatory agents; hormones such as steroids; growth factors, including bone morphogenic proteins (i.e. BMP's 1-7), bone morphogenic-like proteins (i.e. GFD-5, GFD-7 and GFD-8), epidermal growth factor (EGF), fibroblast growth factor (i.e. FGF 1-9), platelet derived growth factor (PDGF), insulin like-growth factor (IGF-I and IGF-II); transforming growth factors (i.e. TGF-beta I-III), vascular endothelial growth factor (VEGF); and other naturally derived or genetically engineered proteins, polysaccharides, glycoproteins, or lipoproteins.

Matrix materials for the present invention may be formulated by mixing one or more therapeutic agents with the polymer. Alternatively, a therapeutic agent could be coated onto the polymer, preferably with a pharmaceutically acceptable carrier. Any pharmaceutical carrier can be used that does not dissolve the polymer. The therapeutic agent may be present as a liquid, a finely divided solid, or any other appropriate physical form. Typically, but optionally, the matrix will include one or more additives, such as diluents, carriers, excipients, stabilizers or the like.

The amount of therapeutic agent will depend on the particular drug being employed and medical condition being treated. Typically, the amount of drug represents about 0.001 percent to about 70 percent, more typically about 0.001 percent to about 50 percent, most typically about 0.001 percent to about 20 percent by weight of the matrix. The quantity and type of polymer incorporated into the drug delivery matrix will vary depending on the release profile desired and the amount of drug employed.

Upon contact with body fluids, the polymer undergoes gradual degradation (mainly through hydrolysis) with concomitant release of the dispersed drug for a sustained or extended period. This can result in prolonged delivery (over, say 1 to 5,000 hours, preferably 2 to 800 hours) of effective amounts (say, 0.0001 mg/kg/hour to 10 mg/kg/hour) of the drug. This dosage form can be administered as is necessary depending on the subject being treated, the severity of the affliction, the judgment of the prescribing physician, and the like. Following this or similar procedures, those skilled in the art will be able to prepare a variety of formulations.

The following examples are illustrative of the principles and practice of this invention, although not limited thereto.

### EXAMPLE OF MANUFACTURE: Forming cable/cover/anchor assemblies In this example, a general ultrasonic welding process was used to form strap, anchor, and cover assembly.

The cover was formed by compression molding (Model 2696, Carver, Inc., Wabash, IN). The material used to form the cover was 95/5 poly(lactide-co-glycolide) (95/5 PLGA) from PURAC (Gorinchem, The Netherlands), with an Inherent Viscosity (I.V.) of 2.33 dl/gm (measured in chloroform at 25°C and a concentration of 0.1 gm/dl). The cover was cut to a square piece with the dimensions of 2 X 4.5 millimeter.

The cable was a three dimensional woven cable made using 95/5 poly(lactide-co-glycolide) (95/5 PLGA) fibers. The fibers are sold under the tradename PANACRYL, (Ethicon, Inc., Somerville, NJ). The cable was 3D woven with 100 Denier fiber and a thickness of 1 millimeter and width of 2 millimeter at Fiber Concepts, Inc. (Conshohocken, PA).

The anchor was made using 95/5 poly(lactide-co-glycolide) by injection molding (Model NN3SM14, Niigata Machinery Plant, Tokyo, Japan).

The anchor was placed into a fixture. The cable was cut to 25 millimeter in length and placed on the anchor by penetrating the cable using the pins of the anchor. The cover is placed to cover the cable. The horn of an ultrasonic welding machine (Model SureWeld 70, Sonobond, West Chester, PA) was applied to the pins and the cover, and melted the pins and the cover. The welding time was set to 2.35 seconds, the holding time was set to 1.5 seconds, and the amplitude was set to 9.

The pullout strength of the assembly was tested. Pullout tests were performed using an Instron 4501 test frame. The anchor was first loaded on a polyurethane foam block with a pre-drilled hole with diameter of 5.1 mm, which was fixed in place by a clamp that allows movement in the X-Y plane but not the Z (pulling) direction. The cable end was held tightly by the grips and then a tensile testing procedure was performed with a cross-head rate of 0.1 millimeter/second. The pullout strength of the control was 12.5 pounds-force (lbf).

### EXAMPLE OF USE: Surgical Procedure A patient is prepared for spinal fusion surgery in a conventional manner. The surgery will fuse one or more levels of the spinal column. The patient is anesthetized in a conventional manner. The tissue repair site is accessed by making an incision through the abdominal cavity and dissecting down to the spinal column. A sterile device of the present invention is prepared for implantation into the patient, the device having anchor members mounted to each end of the cable. The operative site is prepared to receive the anchor members of the repair device by dissecting through the ligamentous structure attached to the vertebral bodies of the spinal column that will be fused. A discectomy procedure is performed to remove the disc of the vertebral level to be fused and a bone graft is inserted into the discs space. A bore hole is drilled into the vertebral body above and below the disc space. The anchor bodies are then inserted into drilled bore holes in the adjoining vertebrae to be fused. The cable of the device is used to prevent migration of the bone graft in order to complete the tissue repair. The incision is approximated in a conventional manner using conventional surgical sutures. The incision is bandaged in a conventional manner, thereby completing the surgical procedure.

The novel devices of the present invention provide the patient and surgeon with multiple advantages. The advantages include increased pull-out strength of the anchor member from the cord.

It should be understood that the embodiments described herein are merely exemplary and that a person skilled in the art may make many variations and modifications without departing from the scope of the present invention. All such variations and modifications are intended to be included within the scope of the invention as defined in the appended claims.

## Claims

1. A deployment stabilization unit (300) for a bone anchoring device (10, 110), comprising:
a post (306) having a proximal end (308) and a distal end (310);
a frangible break-away section (312); and
a stabilization seat (314);
wherein the break-away section (312) connects an end (310) of the post (306) to the stabilization seat (314) such that the post (306) and the stabilization seat (314) may be effectively separated upon the application of a sufficient force;
**characterised by** a seat attachment pin (320) extending out from the stabilization seat (314).

2. A bone anchoring kit comprising a deployment stabilization unit (300) in accordance with claim 1, in combination with a bone anchoring device (10, 110), the bone anchoring device (10, 110) comprising:
a cable member (50, 160) having a first end (52, 162) and a second end (54, 164);
an anchor member (20, 120);
an axial cutout (30, 130) in the anchor member (20, 120) having a surface (32, 132); and
at least one attachment pin (40, 150) extending from the cutout surface (32, 132),
wherein at least a section (58) of the cable member (50, 160) is mounted in the axial cutout (30, 130) adjacent to the surface (32, 132) such that said attachment pin (40, 150) extends through said section (58).

3. The bone anchoring kit of Claim 2, wherein the cable member (50, 160) is selected from the group consisting of solid polymer ribbons, perforated polymer ribbons, braided ribbons, and woven ribbons.

4. The bone anchoring kit of Claim 2 or Claim 3, wherein the distal end (54, 164) of the cable member (50, 160) is encapsulated in a material.

5. The bone anchoring kit of any of Claims 2, 3 and 4, comprising more than one attachment pin (40, 150).

6. The bone anchoring kit of any of Claims 2 to 5, wherein said attachment pin (40, 150) extends perpendicularly from the cutout surface (32, 132).

7. The bone anchoring kit of any of Claims 2 to 6, wherein the device (10, 110) comprises a polymer selected from the group consisting of aliphatic polyester polymer, aliphatic polyester copolymers, and blends thereof.

8. The bone anchoring kit of Claim 7, wherein the aliphatic polyesters comprise monomers selected from the group consisting of lactic acid, lactide, glycolic acid, glycolide, epsilon-caprolactone, 1,4-dioxan-2-one, and 1,3-dioxan-2-one.

9. A bone anchoring kit comprising a deployment stabilization unit (300) in accordance with claim 1, in combination with a bone anchoring device (110), the bone anchoring device (110) comprising:
a cable member (160);
an expander member (180);
an expandable anchor member (120);
an axial passageway (134) in the anchor member (120);
an axial slot (140) in the anchor member (120),
an axial cutout (130) in the anchor member (120) having a surface (132); and,
at least one attachment pin (150) extending from the surface (132),
wherein at least a section (50) of the cable member (160) is mounted in the axial cutout (130) adjacent to the surface (132) such that each attachment pin (150) extends through said section (58).

10. The bone anchoring kit of Claim 9, wherein the cable member (160) is selected from the group consisting of solid polymer ribbons, perforated polymer ribbons, braided ribbons, and woven ribbons.

11. The bone anchoring kit of Claim 9 or Claim 10, wherein the distal end (164) of the cable member is encapsulated in a material.

12. The bone anchoring kit of any of Claims 9, 10 and 11, comprising more than one attachment pin (150).

13. The bone anchoring kit of any of Claims 9, 10, 11 and 12, wherein the expandable anchor member (160) further comprises a wall hinge (142).

14. The bone anchoring kit of any of Claims 9, 10, 11, 12 and 13, wherein the axial passageway (134) has a chamfered or beveled edge.

15. The bone anchoring kit of any of Claims 9 to 14, wherein the expander member (180) additionally comprises a distal end (187) with a chamfered or beveled edge (184).

16. The bone anchoring kit of any of Claims 9 to 15, wherein each attachment pin (150) extends perpendicularly from the cutout surface (132).

17. The bone anchoring kit of any of Claims 9 to 16, wherein the device (110) comprises a polymer selected from the group consisting of aliphatic polyester polymers, aliphatic polyester copolymers, and blends thereof.

18. The bone anchoring kit of Claim 17, wherein the aliphatic polyesters comprise monomers selected from the group consisting of lactic acid, lactide, glycolic acid, glycolide, epsilon-caprolactone, 1,4-dioxan-2-one, and 1,3-dioxan-2-one.

## Patentansprüche

1. Stabilisierungseinheit (300) für das Anbringen einer Knochenverankerungsvorrichtung (10, 110), die aufweist:
einen Zapfen (306) mit einem proximalen Ende und einem distalen Ende (310);
einen zerbrechlichen, wegzubrechenden Abschnitt (312); und
eine Stabilisierungsauflage (314);
wobei der wegzuberechende Abschnitt (312) ein Ende (310) des Zapfens (306) mit der Stabilisierungsauflage (314) verbindet, so dass der Zapfen (306) und die Stabilisierungsauflage (314) beim Anwenden einer ausreichenden Kraft wirksam getrennt werden können;
**gekennzeichnet durch** einen Auflagebefestigungsstift (320), der sich aus der Stabilisierungsauflage (314) heraus erstreckt.

2. Basisausrüstung für die Knochenverankerung, welche eine Stabilisierungseinheit (300) für das Anbringen gemäß Anspruch 1 aufweist, in Kombination mit einer Knochenverankerungsvorrichtung (10, 110), wobei die Knochenverankerungsvorrichtung (10, 110) aufweist:
ein Kabelelement (50, 160) mit einem ersten Ende (52, 162) und einem zweiten Ende (54, 164);
ein Ankerelement (20, 120);
einen axialen Ausschnitt (30, 130) in dem Ankerelement (20, 120), der eine Fläche (32, 132) hat; und wenigstens einen Befestigungsstift (40, 150), der sich aus der Fläche (32, 132) des Ausschnitts erstreckt,
wobei wenigstens ein Abschnitt (58) des Kabelelementes (50, 160) in dem axialen Ausschnitt (30, 130) angrenzend an die Fläche (32, 132) angebracht ist, so dass sich der Befestigungsstift (40, 150) durch den Abschnitt (58) erstreckt.

3. Basisausrüstung zur Knochenverankerung nach Anspruch 2, bei der das Kabelelement (50, 160) aus der Gruppe bestehend aus massiven Polymerbändem, perforierten Polymerbändem, geflochtenen Bändern und gewebten Bändern ausgewählt ist.

4. Basisausrüstung für die Knochenverankerung nach Anspruch 2 oder Anspruch 3, bei der das distale Ende (54, 164) des Kabelelementes (50, 160) in einem Material verkapselt ist.

5. Basisausrüstung zur Knochenverankerung nach einem der Ansprüche 2, 3 und 4, mit mehr als einem Befestigungsstift (40, 150).

6. Basisausrüstung zur Knochenverankerung nach einem der Ansprüche 2 bis 5, bei der der Befestigungsstift (40, 150) sich senkrecht von der Fläche (32, 132) des Ausschnitts erstreckt.

7. Basisausrüstung für die Knochenverankerung nach einem der Ansprüche 2 bis 6, bei der die Vorrichtung (10, 110) ein Polymer aufweist, das aus der Gruppe bestehend aus aliphatischem Polyester-Polymer, aliphatischen Polyester-Copolymeren und Mischungen aus diesen ausgewählt ist.

8. Basisausrüstung für die Knochenverankerung nach Anspruch 7, bei der die aliphatischen Polymere Monomere aufweisen, die aus der Gruppe bestehend aus Milchsäure, Laktid, Glykolsäure, Glykolid, Epsilon-Caprolacton, 1,4-Dioxan-2-on und 1,3-Dioxan-2-on ausgewählt sind.

9. Basisausrüstung für die Knochenverankerung, die eine Stabilisierungseinheit (300) zum Anbringen nach Anspruch 1 aufweist, in Kombination mit einer Knochenverankerungsvorrichtung (110), wobei die Knochenverankerungsvorrichtung (110) aufweist:
ein Kabelelement (160);
ein Aufweiteelement (180);
ein aufweitbares Ankerelement (120);
einen axialen Durchlass (134) in dem Ankerelement (120);
einen axialen Schlitz (140) in dem Ankerelement (120);
einen axialen Ausschnitt (130) in dem Ankerelement (120), der eine Fläche (132) hat;
und
wenigstens einen Befestigungsstift (150), der sich aus der Fläche (132) erstreckt,
wobei wenigstens ein Abschnitt (50) des Kabelelementes (160) in dem axialen Ausschnitt (130) benachbart der Fläche (132) derart angebracht ist, dass sich jeder Befestigungsstift (150) durch den Abschnitt (58) erstreckt.

10. Basisausrüstung für die Knochenverankerung nach Anspruch 9, bei der das Kabelelement (160) aus der Gruppe bestehend aus massiven Polymerbändern, perforierten Polymerbändem, geflochtenen Bändern und gewebten Bändern ausgewählt ist.

11. Basisausrüstung für die Knochenverankerung nach Anspruch 9 oder Anspruch 10, bei der das distale Ende (164) des Kabelelementes in einem Material verkapselt ist.

12. Basisausrüstung für die Knochenverankerung nach einem der Ansprüche 9, 10 und 11, die mehr als einen Befestigungsstift (150) aufweist.

13. Basisausrüstung für die Knochenverankerung nach einem der Ansprüche 9, 10, 11 und 12, bei der das aufweitbare Ankerelement (160) weiter einen Aufhängeschuh (142) aufweist.

14. Basisausrüstung für die Knochenverankerung nach einem der Ansprüche 9, 10, 11, 12 und 13, bei der der axiale Durchlass (134) eine abgeschrägte oder abgefaste Kante hat.

15. Basisausrüstung für die Knochenverankerung nach einem der Ansprüche 9 bis 14, bei der das Aufweiteelement (180) zusätzlich ein distales Ende (187) mit einer abgeschrägten oder abgefasten Kante (184) aufweist.

16. Basisausrüstung für die Knochenverankerung nach einem der Ansprüche 9 bis 15, bei der sich jeder Befestigungsstift (150) senkrecht von der Fläche (132) des Ausschnitts erstreckt.

17. Basisausrüstung für die Knochenverankerung nach einem der Ansprüche 9 bis 16, bei der die Vorrichtung (110) ein Polymer aufweist, das aus der Gruppe bestehend aus aliphatischen Polyester-Polymeren, aliphatischen Polyester-Copolymeren und Mischungen aus diesen ausgewählt ist.

18. Basisausrüstung für die Knochenverankerung nach Anspruch 17, bei der die aliphatischen Polymere Monomere aufweisen, die aus der Gruppe bestehend aus Milchsäure, Laktid, Glykolsäure, Glykolid, Epsilon-Caprolacton, 1,4-Dioxan-2-on und 1,3-Dioxan-2-on besteht.

## Revendications

1. Unité de stabilisation de déploiement (300) pour un dispositif d'ancrage d'os (10, 110), comprenant :
■ un montant (306) ayant une extrémité proximale (308) et une extrémité distale (310) ;
■ une section détachable frangible (312) ; et
■ une assise de stabilisation (314) ;
dans laquelle la section détachable (312) relie une extrémité (310) du montant (306) à l'assise de stabilisation (314), de telle sorte que le montant (306) et l'assise de stabilisation (314) puissent être séparés de manière efficace lors de l'application d'une force suffisante ;
**caractérisée par** une cheville de fixation d'assise (320) s'étendant vers l'extérieur à partir de l'assise de stabilisation (314).

2. Kit d'ancrage d'os comprenant une unité de stabilisation de déploiement (300) selon la revendication 1, en combinaison avec un dispositif d'ancrage d'os (10, 110), le dispositif d'ancrage d'os (10, 110) comprenant :
■ un élément de câble (50, 160) ayant une première extrémité (52, 162) et une seconde extrémité (54, 164) ;
■ un élément d'ancrage (20, 120) ;
■ une découpe axiale (30, 130) dans l'élément d'ancrage (20, 120) ayant une surface (32, 132) ; et
■ au moins une cheville de fixation (40, 150) s'étendant à partir de la surface de découpe (32, 132),
dans laquelle au moins une section (58) de l'élément de câble (50, 160) est montée dans la découpe axiale (30, 130) adjacente à la surface (32, 132) de telle sorte que ladite cheville de fixation (40, 150) s'étende à travers ladite section (58).

3. Kit d'ancrage d'os selon la revendication 2, dans lequel l'élément de câble (50, 160) est choisi dans le groupe comprenant rubans de polymère solides, rubans de polymère perforés, rubans tressés et rubans tissés.

4. Kit d'ancrage d'os selon la revendication 2 ou la revendication 3, dans lequel l'extrémité distale (54, 164) de l'élément de câble (50, 160) est encapsulée dans un matériau.

5. Kit d'ancrage d'os selon l'une quelconque des revendications 2, 3 et 4, comprenant plusieurs chevilles de fixation (40, 150).

6. Kit d'ancrage d'os selon l'une quelconque des revendications 2 à 5, dans lequel ladite cheville de fixation (40, 150) s'étend perpendiculairement à partir de la surface de découpe (32, 132).

7. Kit d'ancrage d'os selon l'une quelconque des revendications 2 à 6, dans lequel le dispositif (10, 110) comprend un polymère choisi dans le groupe comprenant polymères de polyester aliphatique, copolymères de polyester aliphatique et mélanges de ceux-ci.

8. Kit d'ancrage d'os selon la revendication 7, dans lequel les polyesters aliphatiques comprennent des monomères choisis dans le groupe comprenant acide lactique, lactide, acide glycolique, glycolide, epsilon-caprolactone, 1,4-dioxan-2-one et 1,3-dioxan-2-one.

9. Kit d'ancrage d'os comprenant une unité de stabilisation de déploiement (300) selon la revendication 1, en combinaison avec un dispositif d'ancrage d'os (110), le dispositif d'ancrage d'os (110) comprenant :
■ un élément de câble (160) ;
■ un élément élargisseur (180) ;
■ un élément d'ancrage élargissable (120) :
■ un passage axial (134) dans l'élément d'ancrage (120) ;
■ une fente axiale (140) dans l'élément d'ancrage (120),
■ une découpe axiale (130) dans l'élément d'ancrage (120) ayant une surface (132) ; et
■ au moins unr cheville de fixation (150) s'étendant à partir de la surface (132),
dans lequel au moins une section (50) de l'élément de câble (160) est montée dans la découpe axiale (130) adjacente à la surface (132) de telle sorte que chaque cheville de fixation (150) s'étende à travers ladite section (58).

10. Kit d'ancrage d'os selon la revendication 9, dans lequel l'élément de câble (160) est choisi dans le groupe comprenant rubans de polymère solides, rubans de polymère perforés, rubans tressés et rubans tissés.

11. Kit d'ancrage d'os selon la revendication 9 ou la revendication 10, dans lequel l'extrémité distale (164) de l'élément de câble est encapsulée dans un matériau.

12. Kit d'ancrage d'os selon l'une quelconque des revendications 9, 10 et 11, comprenant plusieurs chevilles de fixation (150).

13. Kit d'ancrage d'os selon l'une quelconque des revendications 9, 10, 11 et 12, dans lequel l'élément d'ancrage élargissable (160) comprend en outre une articulation de paroi (142).

14. Kit d'ancrage d'os selon l'une quelconque des revendications 9, 10, 11, 12 et 13, dans lequel le passage axial (134) a un bord chanfreiné ou biseauté.

15. Kit d'ancrage d'os selon l'une quelconque des revendications 9 à 14, dans lequel l'élément élargisseur (180) comprend en outre une extrémité distale (187) avec un bord chanfreiné ou biseauté (184).

16. Kit d'ancrage d'os selon l'une quelconque des revendications 9 à 15, dans lequel chaque cheville de fixation (150) s'étend perpendiculairement à partir de la surface de découpe (132).

17. Kit d'ancrage d'os selon l'une quelconque des revendications 9 à 16, dans lequel le dispositif (110) comprend un polymère choisi dans le groupe comprenant polymères de polyester aliphatique, copolymères de polyester aliphatique et mélanges de ceux-ci.

18. Kit d'ancrage d'os selon la revendication 17, dans lequel les polyesters aliphatiques comprennent des monomères choisis dans le groupe comprenant acide lactique, lactide, acide glycolique, glycolide, ε-caprolactone, 1,4-dioxan-2-one et 1, 3-dioxan-2-one.
